# EUROPEAN PATENT APPLICATION

(11) **EP 4 119 195 A1**
(43) Date of publication of application: **18.01.2023**
(21) Application number: 21767506.5
(22) Date of filing: 09.03.2021
(51) Int. Cl.: A61Q 1/12, A61K 8/19, A61K 8/36, A61K 8/37, A61K 8/44, A61K 8/891

(54) **SOLID POWDER COSMETIC**

(30) Priority: 09.03.2020 JP 2020040081
(71) Applicant: AJINOMOTO CO., INC., Chuo-ku Tokyo 104-8315 (JP)
(72) Inventor: IKEDA Shiori, Kawasaki-shi, Kanagawa 210-8681 (JP)
(74) Representative: Mewburn Ellis LLP
(86) International application number: PCT/JP2021/009315
(87) International publication number: WO 2021/182473

(57) **Abstract**

The purpose of the present invention is to provide a solid powder cosmetic that achieves softness and ease of puff uptake as well as formability and strength (impact resistance). The present invention is a solid powder cosmetic comprising (A) an acyl lysine, (B) a fatty acid metal salt, (C) an oil agent or oil agent mixture that is liquid at 25°C, and (D) an inorganic powder. The total content of component (A) and component (B) is 10-40 mass% of the overall solid powder cosmetic; the content of component (C) is 1-6 mass% of the overall solid powder cosmetic; and the contents of components (A), (B), and (C) satisfy the relationship ((A) + (B)) × (C) = 40-150.

## Description

### Technical Field

The present invention relates to a solid powder cosmetic.

### Background Art

To impart formability to a solid powder cosmetic, an approach of increasing the amount of oil agent blended has conventionally been employed. However, when an oil agent is blended in such an amount that a sufficient formability can be obtained, there arise problems such as hardening of the cake and difficulty in taking in the solid powder cosmetic with a puff. In general, improvement in feel in use such as softness and ease of intake by a puff usually sacrifices not only formability but also the strength (impact resistance).

Japanese Patent Application Publication No. 2013-209346 discloses a solid powder cosmetic that is excellent in formability and impact resistance, offers a smooth feel during application, is excellent in adhesion to the skin, has a reduced powdery feel, and conceals irregularities such as skin pores. However, the solid powder cosmetic is embodied by using a fluorine-treated powder, and there remains a problem related to an embodiment using am untreated powder, which is less expensive.

Meanwhile, the powder cosmetic disclosed in Japanese Patent Application Publication No. 2017-197496 still has a problem in terms of achieving softness and impact resistance simultaneously.

### Citation List

### Patent Literatures

Patent Literature 1: Japanese Patent Application Publication No. 2013-209346
Patent Literature 2: Japanese Patent Application Publication No. 2017-197496

### Summary of Invention

An object of the present invention is to provide a solid powder cosmetic that simultaneously achieves softness and ease of intake by a puff, as well as formability and strength (impact resistance).

The present inventors have made intensive studies, and consequently have succeeded in preparing a soft cake without impairing formability by reducing the amount of oil agents blended and blending a large amount of an acyl lysine. In addition, the use of a fatty acid metal salt in combination and blending of these components at an optimum blending ratio made it possible to achieve ease of intake by a puff, softness, and strength (impact resistance) simultaneously. Moreover, adjustment of the amounts of the components blended enabled improvement in wet feel in use. Specifically, the present invention provides the following:
[1] A solid powder cosmetic, including:
   (A) an acyl lysine;
   (B) a fatty acid metal salt;
   (C) an oil agent or oil agent mixture that is liquid at 25°C; and
   (D) an inorganic powder, in which
   the total amount of the component (A) and the component (B) is 10 to 40% by mass of the entire solid powder cosmetic,
   the amount of the component (C) is 1 to 6% by mass of the entire solid powder cosmetic, and
   the amounts of the components (A) to (C) satisfy a relationship of ((A)+(B))×(C)=40 to 150.
[2] The solid powder cosmetic according to [1], in which
   the mass ratio ((A)/(B)) of the component (A) to the component (B) is 1 to 15.
[3] The solid powder cosmetic according to [1] or [2], in which
   the component (A) is lauroyl lysine.
[4] The solid powder cosmetic according to any one of [1] to [3], further comprising (E) a spherical powder at 2 to 10% by mass.
[5] The solid powder cosmetic according to any one of [1] to [4], in which
   the component (B) is one or two selected from myristic acid metal salts and stearic acid metal salts.
[6] The solid powder cosmetic according to any one of [1] to [5], in which
   the component (C) has a viscosity of 180 mPa to 3000 mPa at 25°C.
[7] The solid powder cosmetic according to any one of [1] to [6], which has a breaking strength of 45 g or higher.

### Brief Description of Drawings

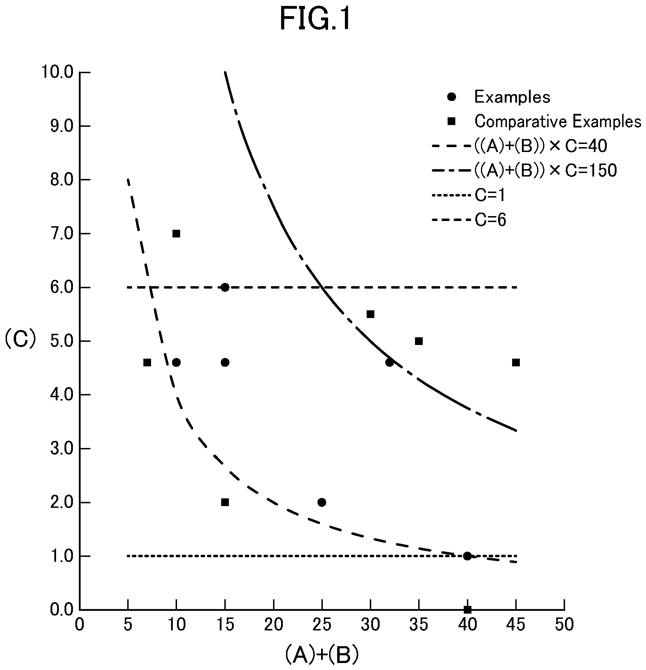

Fig. 1 is a graph in which the relationship between (A)+(B) and (C) is plotted for solid powder cosmetics obtained in Examples and Comparative Examples.

### Description of Embodiments

A solid powder cosmetic of the present invention comprises the following components (A) to (D):
(A) an acyl lysine;
(B) a fatty acid metal salt;
(C) an oil agent or oil agent mixture that is liquid at 25°C; and
(D) an inorganic powder.

The acyl component constituting the acyl group of the acyl lysine serving as the component (A) may be an acyl group that is derived or derivable from a linear or branched and saturated or unsaturated fatty acid, and examples thereof include single-fatty acid acyl groups such as octanoyl, caproyl, nonanoyl, caprinoyl, decanoyl, undecanoyl, lauroyl, myristoyl, palmitoyl, stearoyl, behenoyl, palmitoleoyl, oleoyl, and linoleoyl groups, and natural mixed fatty acid acyl groups such as coconut oil fatty acid acyl and hardened beef tallow fatty acid acyl, as well as aromatic carboxylic acid acyl groups such as a benzoic acid acyl group, and the like. Such acyl groups can be derived from fatty acids, and also can be derived from raw material substances (fatty acid esters, fatty acid salts, acid halides, and acid anhydrides) other than fatty acids, as well. The acyl group is preferably a lauroyl group or an octanoyl group and is more preferably a lauroyl group.

The acyl lysine is preferably an N-acyl lysine having a carbon chain length of C8 to C20 and is more preferably an N-acyl lysine having a carbon chain length of C14 to C18.

In addition, it is only necessary that at least one amino group be acylated, and a form of a mono-N-acyl derivative is preferable. The component (A) is more preferably Nε-acyl-L-lysine.

The amount of the component (A) contained in the solid powder cosmetic is preferably 5 to 30% by mass, and more preferably 5 to 20% by mass.

The fatty acid component constituting the fatty acid metal salt serving as component (B) may be any linear or branched and saturated or unsaturated fatty acid. Examples thereof include lauric acid, myristic acid, palmitic acid, stearic acid, isostearic acid, ricinoleic acid, and the like, and myristic acid or stearic acid is preferable.

The metal constituting the fatty acid metal salt may be any of alkali metals such as sodium and potassium and divalent or trivalent non-alkali metals. Examples thereof include calcium, magnesium, lithium, barium, zinc, and the like.

Fatty acid metal salts include sodium stearate, potassium laurate, lithium stearate, magnesium stearate, calcium stearate, aluminum stearate, barium stearate, zinc stearate, calcium laurate, barium laurate, zinc laurate, magnesium myristate, calcium ricinoleate, and barium ricinoleate.

The amount of the component (B) contained in the solid powder cosmetic is preferably 2 to 10% by mass, and more preferably 5 to 10% by mass.

The total amount of the component (A) and the component (B) contained in the solid powder cosmetic of the present invention is 10 to 40% by mass, preferably 10 to 35% by mass, more preferably 10 to 32% by mass, and more preferably 10 to 30% by mass of the entire solid powder cosmetic. When the total amount of the component (A) and the component (B) is within such a range, the obtained solid powder cosmetic can be excellent in softness (feel in use) and also can have a sufficient strength.

In addition, the mass ratio ((A)/(B)) of the component (A) to the component (B) in the solid powder cosmetic of the present invention is preferably 1 to 15, and more preferably 2 to 15. When the mass ratio of the component (A) to the component (B) is within such a range, the solid powder cosmetic can be excellent in softness (feel in use) and wet feel, and also can have a sufficient strength.

The oil agent as the component (C) is not particularly limited, as long as the oil agent is liquid at 25°C, or when multiple oil agent components are used, the oil agent mixture is liquid at 25°C. Examples of the oil agent as the component (C) include higher alcohols such as octyldodecanol and oleyl alcohol; hydrocarbon oils such as squalane, liquid paraffin, hydrogenated polyisobutene, and isododecane; natural or synthetic ester oils such as jojoba seed oil, isononyl isononanoate, isostearyl neopentanoate, cetyl 2-ethylhexanoate, ethylhexyl palmitate, alkyl benzoates, polyglyceryl-2 tetraisostearate, (phytosteryl/octyldodecyl) lauroyl glutamate, isopropyl lauroyl sarcosinate, ethylhexyl methoxycinnamate, (phytosteryl/decyltetradecyl) myristoyl methyl beta-alaninate, and glyceryl caprate; diglycerides; natural or synthetic triglycerides such as corn oil, olive oil, sunflower oil, glyceryl tri(caprinate/caprylate), and triethylhexanoin; high-viscosity oils such as (phytosteryl/isostearyl/cetyl/stearyl/behenyl) dimer dilinoleate, diisostearyl malate, hydrogenated polydecene, polyglyceryl-2 triisostearate, polyglyceryl-2 diisostearate, and pentaerythrityl tetraisostearate; silicone oils such as dimethicone, methicone, cyclopentasiloxane, cyclohexasiloxane, phenyl trimethicone, and PEG-10 dimethicone; fluorine-containing oil agents such as perfluoropolyether, perfluorodecalin, and perfluorooctane; mineral oils; and the like. One of these oil agents may be used alone, or two or more thereof may be used in combination.

The viscosity of the component (C) at 25°C is preferably 180 mPa to 3000 mPa. When multiple components (C) are contained, the viscosity after mixing is preferably within this range. When such an oil agent is contained, a powder layer and an oil layer are well mixed with each other, so that a solid powder cosmetic excellent in impact resistance can be obtained. The viscosity at 25°C can be measured by a known method, and, for example, can be determined by using a B-type viscometer and selecting a suitable rotor.

The amount of the component (C) contained in the solid powder cosmetic is 1 to 6% by mass, and preferably 1 to 5% by mass. When the amount of the component (C) contained in the solid powder cosmetic is within such a range, the obtained solid powder cosmetic can be excellent in formability and also can have a soft feel.

The amounts of the components (A) to (C) contained in the solid powder cosmetic of the present invention satisfy a relationship of ((A)+(B))×(C)=40 to 150. The value of ((A)+(B))×(C) is preferably 50 to 150. When the value of ((A)+(B))×(C) is within such a range, the obtained solid powder cosmetic can be excellent in softness (feel in use) and also can have a wet feel and a sufficient strength.

Examples of the inorganic powder serving as the component (D) include yellow iron oxide, red iron oxide, black iron oxide, iron oxide fine particles, bismuth oxychloride, zirconium oxide, magnesium oxide, chromium oxide, cobalt oxide, carbon black, ultramarine, Prussian blue, zinc oxide, zinc oxide fine particles, titanium oxide, titanium oxide fine particles, silica, porous silica, alumina, cerium oxide, boron nitride, calcium sulfate, barium sulfate, magnesium sulfate, calcium carbonate, magnesium carbonate, aluminum silicate, magnesium silicate, silicon carbide, dyes, lakes, sericite, mica, talc, kaolin, clay, bentonite, plate-shaped barium sulfate, butterfly-shaped barium sulfate, hydroxyapatite, and the like. One of these inorganic powders may be used alone, or two or more thereof may be used in combination. Furthermore, the inorganic powder may be a composite material of any of the above-described inorganic powders (for example, silica-coated titanium oxide, mica-coated titanium oxide, or titanium-coated mica), or may be one obtained by subjecting any of the above-described inorganic powders to a surface treatment such as a silicone treatment, a fluorine-containing compound treatment, a silane coupling agent treatment, a silane treatment, an organic titanate treatment, a fatty acid treatment (for example, a stearoyl glutamic acid treatment), a metallic soap treatment (for example, an aluminium stearate treatment), an oil agent treatment, or an amino acid treatment (for example, silicone-treated talc, silicone-treated mica, silicone-treated sericite, silicone-treated titanium oxide, silicone-treated red iron oxide, silicone-treated yellow iron oxide, silicone-treated black iron oxide, stearoyl glutamic acid-treated titanium oxide, stearoyl glutamic acid-treated yellow iron oxide, stearoyl glutamic acid-treated red iron oxide, stearoyl glutamic acid-treated black iron oxide, aluminium stearate-treated titanium oxide, or the like). Since a cosmetic composition of the present invention containing talc as the component (D) provides an effect of the present invention more remarkably, it is preferable to use talc as the component (D).

The solid powder cosmetic of the present invention may further comprise a spherical powder as a component (E). Note, however, that those regarded as the component (D) are excluded from the component (E). The spherical powder is a component for improvement in spreadability during application. Specific examples of the spherical powder include silicic acid anhydride, polymethyl methacrylate, nylon, cross-linked silicone, and the like, and one or two or more thereof may be used.

The amount of the component (E) contained in the solid powder cosmetic is preferably 2 to 10% by mass, and more preferably 3 to 9% by mass. When such an amount of the component (E) is contained in the solid powder cosmetic, moderate voids are created in the cosmetic, improving the ease of intake by a puff.

The solid powder cosmetic containing the above-described components has a breaking strength of preferably 45 g or higher, and more preferably 50 g or higher. A solid powder cosmetic having such a breaking strength is resistant to breakage when falling down.

The solid powder cosmetic of the present invention can be used in various applications for the purpose of color control or coloring of the skin or the lips or shielding from ultraviolet rays. The solid powder cosmetic of the present invention may be, for example, a solid powder cosmetic formed by packing in a container followed by compression molding or solvent removal, or the like. Applications of the solid powder cosmetic of the present invention include foundations, stick foundations, Oshiroi (a powder foundation), lipsticks, cheek rouge, colored eye shadow, eyebrow pencils, makeup bases, daytime cosmetic serums, sunscreens, concealers, bronzers, colored lipsticks, and BB creams. Characteristics of the solid powder cosmetic of the present invention are particularly made good use of in applications where a relatively large amount of an inorganic powder is blended, and examples of solid powder cosmetics used for such applications include solid powder cosmetics such as solid powder foundations, powder cosmetics such as loose powder foundations and Oshiroi, various sunscreens, concealers, makeup bases, and the like.

In addition to the above-described components (A) to (E), additional components commonly usable in cosmetics (including topical medications and quasi drugs) may be blended in the solid powder cosmetic of the present invention, unless an effect of the present invention is impaired. Examples of the additional components include water, surfactants, amino acids, amino acid derivatives, lower alcohols (for example, ethanol), polyols (for example, glycerin, butylene glycol), sugar alcohols and alkylene oxide adducts thereof, water-soluble polymers (for example, hydroxyethyl cellulose), film-forming polymers, gelling agents (for example, dibutyl lauroyl glutamide, dibutyl ethylhexanoyl glutamide), humectants (for example, sodium pyrrolidonecarboxylate), microbicides and antibacterial agents, anti-inflammatory agents, analgesic agents, antifungal agents, agents for softening and exfoliating the stratum corneum, skin-coloring agents, hormone preparations, ultraviolet absorbers, hair growth agents, antiperspirant agents and astringent components (for example, pyrrolidonecarboxylic acid zinc salt), perspiration deodorants, vitamin preparations, blood flow-enhancing agents (vasodilators, blood circulation promoters), herbal medicines, plant extracts, pH-adjusting agents, chelating agents (for example, EDTA-2Na), viscosity modifiers, pearling agents, natural flavors and/or fragrances, synthetic flavors and/or fragrances, dyes and pigments (for example, Red 202, Blue 1), antioxidants (for example, tocopherols, tocopheryl acetates, pentagalloyl glucoside), preservatives (for example, methyl paraben, butyl paraben, propyl paraben, phenoxyethanol), emulsifiers, thickeners, fats and waxes, silicone compounds, perfume oils, and the like.

The solid powder cosmetic of the present invention can be produced by mixing the components (A) to (E) and, if required, additional components together by a known method. For example, the solid powder cosmetic of the present invention can be produced by adding the component (C) and, if required, additional components to the components (A), (B), and (D), which are powders, and further optionally the component (E), then mixing them with each other by using a Henschel mixer or the like, and sieving the obtained mixture. The solid powder cosmetic of the present invention can also be produced by coating a portion or all of the component (D) with a portion or all of the component (A) by a known method, and then mixing the component (D) coated with the component (A) with the remaining portion of the component (A) (when a portion of the component (A) is used for the coating), the remaining portion of the component (D) (when a portion of the component (D) is coated), and the components (B) and (C), and, if required, additional components. The method for coating the component (D) with the component (A) may be a dry treatment method using an impact mixer, a shear mixer, or the like, or a wet treatment method in which a strong alkaline solution of the component (A) is added dropwise to a slurry of the component (D) followed by neutralization with an acid, filtration, and drying. When the component (D) is coated with the component (A), the amount of the component (A) used for the coating is preferably 20 parts by weight or less relative to 100 parts by weight of the component (D) to be coated.

Next, the present invention is described based on Examples and Comparative Examples; however, the present invention is not limited thereto.

### [Examples]

### <Examples 1 to 6 and Comparative Examples 1 to 7>

The powder components shown in Table 1 were mixed by using a food processor, and further the oil agent components were added followed by mixing. The obtained mixture was passed through a 149 µm mesh sieve, and evaluated in terms of the formability, strength (impact resistance), softness, intake, and wet feel as described below. Table 1 shows the results.

### <Viscosity of Oils>

The components (C) were mixed and measured for 30 seconds by using a B-type viscometer: DVL-B (manufactured by TOKYO KEIKI INC.) in a chamber set at 25°C, while using a No.2 or No.4 rotor according to the range of viscosity of the mixture to be measured at a number of revolutions of 30 rpm.

### <Evaluations>

### (1) Evaluation of Formability

A solid powder cosmetic was prepared by compression molding into a size of 2×4 cm with a force of 0.4 mPa. Whether the solid powder cosmetic retained the solid shape when detached from the mold was observed.

### <Evaluation Criteria>

A: Retain
D: Collapse

### (2) Evaluation of Strength (Impact Resistance)

A solid powder cosmetic was prepared by compression molding into a size of 2×4 cm with a force of 0.6 mPa, and measured for a load required for break (breaking strength) by using a rheometer manufactured by FUDOH rheometer. On the basis of the obtained measured values, evaluation was made by using the following evaluation criteria.

### <Evaluation Criteria>

A: The load (breaking strength) is 50 g or higher
B: The load (breaking strength) is 45 g or higher and lower than 50 g
C: The load (breaking strength) is 40 g or higher and lower than 45 g
D: The load (breaking strength) is lower than 40 g or unmeasurable

### (3) Evaluation of Softness

A solid powder cosmetic was prepared in a Φ58 circular metal dish by compression molding with a force of 0.6 mPa, and measured for its hardness by a needle penetration test using a rubber durometer manufactured by TECLOCK corporation. On the basis of the obtained measured values, evaluation was made using the following evaluation criteria.

### <Evaluation Criteria>

A: The hardness is lower than 13
B: The hardness is 13 or higher and lower than 16
C: The hardness is 16 or higher and lower than 20
D: The hardness is 20 or higher

### (4) Evaluation of Intake

A solid powder cosmetic was prepared in a Φ58 circular metal dish by compression molding with a force of 0.6 mPa, and measured for the amount of the solid powder cosmetic taken in by a commercially available foundation puff when the foundation puff was moved back and forth twice by using a device for measuring kinetic friction manufactured by Trinity-Lab inc. On the basis of the obtained measured values, evaluation was made using the following evaluation criteria.

### <Evaluation Criteria>

A: The weight change is 7.0 mg or more
B: The weight change is 6.0 mg or more and less than 7.0 mg
C: The weight change is 5.0 mg or more and less than 6.0 mg
D: The weight change is less than 5.0 mg

### (5) Evaluation of Wet Feel

Five professional panelists took each of the solid powder cosmetics of Examples by the hand and applied it onto the back of the other hand. Here, the wet feel was evaluated by the panelists based on the sensory evaluation criteria described below and scored. The sensory evaluation scores given by the five professional panelists were summed up, and the wet feel was evaluated on the basis of the evaluation criteria described below.

### <Sensory Evaluation Criteria>

A: Definitely has a wet feel
B: Has a slight wet feel
C: Slightly powdery
D: Definitely powdery

### (6) Overall Evaluation

For each of the evaluation items: formability, strength (impact resistance), softness, and intake, five points were allotted to evaluation A, two points were allotted to evaluation B, one point was allotted to evaluation C, and zero points were allotted to evaluation D. Then, the total of the evaluation scores was determined. If the overall score was 17 points or higher, the solid powder cosmetic was regarded as Example.

**Table 1**

| | Components (Parts by Mass) | Ex. 1 | Ex. 2 | Ex. 3 | Ex. 4 | Ex. 5 | Ex. 6 |
|---|---|---|---|---|---|---|---|
| (A) | Nε-Lauroyl Lysine | 10 | 30 | 5 | 30 | 10 | 20 |
| (B) | Magnesium Myristate | 5 | 2 | 5 | 10 | 5 | 5 |
| (C) | Octyldodecyl Stearoyl Stearate | 1.6 | 1.6 | 1.6 | 0.37 | 2.2 | 0.74 |
| | Ethylhexyl Palmitate | 0.8 | 0.8 | 0.8 | 0.16 | 1 | 0.32 |
| | Hydrogenated Polyisobutene | 0.8 | 0.8 | 0.8 | 0.16 | 1 | 0.32 |
| | Dimethicone | 0.8 | 0.8 | 0.8 | 0.16 | 1 | 0.32 |
| | Pentaerythrityl Tetra(2-ethylhexanoate) | 0.4 | 0.4 | 0.4 | 0.1 | 0.6 | 0.2 |
| | PEG-15 Glyceryl Isostearate | 0.2 | 0.2 | 0.2 | 0.05 | 0.2 | 0.1 |
| (D) | Talc | 69.4 | 52.4 | 74.4 | 48 | 68 | 62 |
| | Other Inorganic Powders such as Pigment 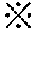 | 5 | 5 | 5 | 5 | 5 | 5 |
| (E) | Nylon | 5 | 5 | 5 | 5 | 5 | 5 |
| | (Diphenyl Dimethicone/Vinyl Diphenyl Dimethicone/Silsesquioxane) | 1 | 1 | 1 | 1 | 1 | 1 |
| | Crosspolymer | | | | | | |
| | Total | 100 | 100 | 100 | 100 | 100 | 100 |
| | (A)+(B) | 15 | 32 | 10 | 40 | 15 | 25 |
| | (A)/(B) | 2.0 | 15.0 | 1.0 | 3.0 | 2.0 | 4.0 |
| | (C) | 4.6 | 4.6 | 4.6 | 1.0 | 6.0 | 2.0 |
| | ((A)+(B))×(C) | 69.0 | 147.2 | 46.0 | 40.0 | 90.0 | 50.0 |
| | Viscosity of Oils | 231 | 231 | 231 | 231 | 231 | 231 |
| | Formability | A | A | A | A | A | A |
| | Strength | A | A | A | A | A | A |
| | Ease of Intake | A | A | A | A | A | A |
| | Softness | A | A | A | A | B | A |
| | Wet Feel | A | B | C | C | C | - |
| | Overall Evaluation | 20 | 20 | 20 | 20 | 17 | 20 |

**Table 2**

| | Component (Parts by Mass) | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|---|
| (A) | Nε-Lauroyl Lysine | 5 | 40 | 5 | 30 | 10 | 25 | 30 |
| (B) | Magnesium Myristate | 2 | 5 | 5 | 10 | 5 | 5 | 5 |
| (C) | Octyldodecyl Stearoyl Stearate | 1.6 | 1.6 | 2.2 | | 0.74 | 2 | 1.9 |
| | Ethylhexyl Palmitate | 0.8 | 0.8 | 1.1 | | 0.32 | 0.9 | 0.8 |
| | Hydrogenated Polyisobutene | 0.8 | 0.8 | 1.1 | | 0.32 | 0.9 | 0.8 |
| | Dimethicone | 0.8 | 0.8 | 1.1 | | 0.32 | 0.9 | 0.8 |
| | Pentaerythrityl Tetra(2-ethylhexanoate) | 0.4 | 0.4 | 1.1 | | 0.2 | 0.55 | 0.5 |
| | PEG-15 Glyceryl Isostearate | 0.2 | 0.2 | 0.4 | | 0.1 | 0.25 | 0.2 |
| (D) | Talc | 77.4 | 39.4 | 72 | 49 | 72 | 53.5 | 49 |
| | Other Inorganic Powders such as Pigment 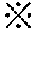 | 5 | 5 | 5 | 5 | 5 | 5 | 5 |
| (E) | Nylon | 5 | 5 | 5 | 5 | 5 | 5 | 5 |
| | (Diphenyl Dimethicone/Vinyl Diphenyl Dimethicone/Silsesquioxane) Crosspolymer | 1 | 1 | 1 | 1 | 1 | 1 | 1 |
| | Total | 100 | 100 | 100 | 100 | 100 | 100 | 100 |
| | (A)+(B) | 7 | 45 | 10 | 40 | 15 | 30 | 35 |
| | (A)/(B) | 2.5 | 8.0 | 1.0 | 3.0 | 2.0 | 5.0 | 6.0 |
| | (C) | 4.6 | 4.6 | 7.0 | 0 | 2.0 | 5.5 | 5.0 |
| | ((A)+(B)) ×(C) | 32.2 | 207.0 | 70.0 | 0 | 30.0 | 165.0 | 175.0 |
| | Viscosity of Oils | 231 | 231 | 231 | 231 | 231 | 231 | 231 |
| | Formability | A | A | A | D | A | A | A |
| | Strength | D | A | A | - | C | A | A |
| | Ease of Intake | A | B | B | A | A | C | B |
| | Softness | A | C | C | A | A | C | C |
| | Wet Feel | - | - | - | - | - | - | - |
| | Overall Evaluation | 15 | 13 | 13 | 10 | 16 | 12 | 13 |

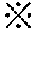 A composition consisting of the following components was used as the "Other inorganic powders such as pigment" in Tables 1 and 2.

| | |
|---|---|
| Boron nitride | 1 part by mass |
| Synthetic phlogopite | 1 part by mass |
| Pigment titanium oxide | 1 part by mass |
| Iron oxide (red) | 0.5 parts by mass |
| Iron oxide (yellow) | 1.2 parts by mass |
| Iron oxide (black) | 0.3 parts by mass |

Components used in Examples and Comparative Examples were as follows:

**Table 3**

| Components | product name | supplier's name |
|---|---|---|
| Nε-Lauroyl Lysine | AMIHOPE LL | AJINOMOTO CO., INC. |
| Magnesium Myristate | Magnesium Myristate | Taihei Chemical Industrial Co., Ltd. |
| Octyldodecyl Stearoyl Stearate | RISOCAST ODSHS | KOKYU ALCOHOL KOGYO CO., LTD. |
| Ethylhexyl Palmitate | SALACOS P-8 | The Nisshin OilliO Group, Ltd. |
| Hydrogenated Polyisobutene | PARLEAM 18 | NOF CORPORATIO N |
| Dimethicone | KF-96A-101cs | Shin-Etsu Silicone |
| Pentaerythrityl Tetra(2-ethylhexanoate) | SALACOS 5409 | The Nisshin OilliO Group, Ltd. |
| PEG-15 Glyceryl Isostearate | GWIS-116 | Nihon Emulsion Co., Ltd. |
| Nylon | SP-10 | Toray Industries, Inc. |
| (Diphenyl Dimethicone/Vinyl Diphenyl Dimethicone/Silsesquioxane) Crosspolymer | KSP-300 | Shin-Etsu Silicone |
| Talc | JA-24R | ASADA MILLING CO.,LTD. |
| Boron Nitride | SHP-5 | Mizushima Ferroalloy Co., Ltd. |
| Synthetic Phlogopite | PFD-8W | Topy Industries, Ltd. |
| Pigment Titanium Oxide | CR-50 | Ishihara Sangyo Kaisha, Ltd. |
| Iron Oxide (Red) | SA-Red | Miyoshi Kasei, Inc. |
| Iron Oxide (Yellow) | SA-Yellow | Miyoshi Kasei, Inc. |
| Iron Oxide (Black) | SA-Black | Miyoshi Kasei, Inc. |

Regarding Comparative Example 1 in which the A+B value was less than 10% of the entire solid powder cosmetic, the strength was poor. Regarding Comparative Example 2 in which the A+B value exceeded 40% of the entire solid powder cosmetic, the softness was poor.

Regarding Comparative Example 4 in which the amount of C was 0, the formability was extremely low, and the shape was not retained. Regarding Example 5 in which the amount of C was 6% by mass, the evaluations of softness and wet feel was slightly poor. It was suggested that the upper limit is preferably about 6%. Regarding Comparative Example 3 in which the amount of C exceeded 6% by mass, the softness was poor. Examples 1 to 3 suggested that the amount of C is more preferably 5% or less. Regarding Examples 1 to 5 in which the ((A)+(B))×(C) value was in the rage of from 40 to 150, it was found that all the evaluations of formability, strength, ease of intake, and softness were excellent. Regarding Examples 1 and 2 in which the ((A)+(B))×(C) value was in the range of from 50 to 150, it was found that the wet feel in use was further excellent.

Note that even when the A+B value is 10 to 40% by mass of the entire solid powder cosmetic, a A/B value not within the range of 1 to 15 may result in loss of the wet feel. The wet feel was higher in Example 1 in which the A/B value was 2 than in Example 3 in which the A/B value was 1. Therefore, it is suggested that when the A+B value is constant, a larger A/B value leads to a better wet feel.

## Claims

1. A solid powder cosmetic, comprising:
(A) an acyl lysine;
(B) a fatty acid metal salt;
(C) an oil agent or oil agent mixture that is liquid at 25°C; and
(D) an inorganic powder, wherein
the total amount of the component (A) and the component (B) is 10 to 40% by mass of the entire solid powder cosmetic,
the amount of the component (C) is 1 to 6% by mass of the entire solid powder cosmetic, and
the amounts of the components (A) to (C) satisfy a relationship of ((A)+(B))×(C)=40 to 150.

2. The solid powder cosmetic according to claim 1, wherein
the mass ratio ((A)/(B)) of the component (A) to the component (B) is 1 to 15.

3. The solid powder cosmetic according to claim 1 or 2, wherein
the component (A) is lauroyl lysine.

4. The solid powder cosmetic according to any one of claims1 to 3, further comprising (E) a spherical powder at 2 to 10% by mass.

5. The solid powder cosmetic according to any one of claims 1 to 4, wherein
the component (B) is one or two selected from myristic acid metal salts and stearic acid metal salts.

6. The solid powder cosmetic according to any one of claims 1 to 5, wherein
the component (C) has a viscosity of 180 mPa to 3000 mPa at 25°C.

7. The solid powder cosmetic according to any one of claims 1 to 6, which has a breaking strength of 45 g or higher.
